# EUROPEAN PATENT SPECIFICATION

(11) **EP 2 755 183 B1**
(45) Date of publication and mention of the grant of the patent: **03.04.2019**
(21) Application number: 11872154.7
(22) Date of filing: 08.09.2011
(51) Int. Cl.: G06F 19/00, G06Q 50/00

(54) **HEALTH CARE SYSTEM BASED ON VIDEO IN REMOTE HEALTH CARE SOLUTION AND METHOD FOR PROVIDING HEALTH CARE SERVICE**
GESUNDHEITSÜBERWACHUNGSSYSTEM AUF VIDEOBASIS BEI EINER ENTFERNTEN GESUNDHEITSDIENSTLÖSUNG UND VERFAHREN ZUR BEREITSTELLUNG DES GESUNDHEITSDIENSTES
SYSTÈME DE SOINS DE SANTÉ BASÉ SUR LA VIDÉO DANS UNE SOLUTION DE SOINS DE SANTÉ À DISTANCE ET PROCÉDÉ PERMETTANT DE FOURNIR UN SERVICE DE SOINS DE SANTÉ

(43) Date of publication of application: 16.07.2014
(73) Proprietor: LG Electronics Inc., Seoul 150-721 (KR)
(72) Inventor: PARK, Myungeun, Seoul 137-900 (KR); HONG, Seungbum, Seoul 139-759 (KR)
(74) Representative: Katérle, Axel
(86) International application number: PCT/KR2011/006679
(87) International publication number: WO 2013/035902

(56) References cited:
- EP-A1- 1 942 798
- EP-A1- 2 066 254
- EP-A2- 2 267 622
- KR-A- 20010 045 558
- KR-A- 20010 093 987
- KR-A- 20100 014 065
- KR-B1- 100 719 160
- US-A1- 2009 030 286
- US-A1- 2009 275 805
- ZAN GAO ET AL: "Multi-camera Monitoring of Infusion Pump Use", SEMANTIC COMPUTING (ICSC), 2010 IEEE FOURTH INTERNATIONAL CONFERENCE ON, IEEE, PISCATAWAY, NJ, USA, 22 September 2010 (2010-09-22), pages 105-111, XP031795795, ISBN: 978-1-4244-7912-2

## Description

### TECHNICAL FIELD

The present disclosure relates to a method of providing health care services through a terminal.

### BACKGROUND ART

In recent years, with an increase of interest in health, studies on health care systems for more effective health care and health devices constituting those health care systems have been continuously carried out. Technologies for health devices capable of checking personal health, such as a fall sensor, a medication sensor, an electrocardiogram sensor and the like into which a blood pressure meter, a blood glucose meter, a body fat analyser, or the like and IT technologies are merged have been continually developed.

Furthermore, in recent years, remote solutions capable of caring for personal health or examining chronically ill patients have been developed. Those solutions allow an individual to directly measure his or her biometric information (for example, blood glucose, blood pressure, body fat, etc.) at home, and transfer the measured results to a healthcare professional (for example, a doctor, a pharmacist or a care coordinator), thereby allowing him or her to remotely treat or care for personal disease and health. Accordingly, the professionals may determine a patient's health status and risk with biometric information values measured by the user, and thus the accuracy and reliability of measurement values are very critical.

WO 2007/049174 A1 relates to a system and method for unsupervised blood pressure monitoring and therapy. Information about the patient's "motor activity" prior to the blood pressure measurement is employed for automatically assessing the quality of the subsequently measured blood pressure values, i.e. the trustworthiness of these values is automatically determined. The motor activity of the patient comprises the physical activity of the patient. According to a specific embodiment, a number of additional auxiliary devices provide for additional measuring-related information, which has been obtained before and/or during the blood pressure measurement, for assessing the blood pressure values.

"Multi-Camera Monitoring of Infusion Pump Use", Zan Gao et al., 2010 IEEE Fourth International Conference on Semantic Computing, pages 105 to 111 relates to observational methods to detect errors in the preparation and operation of a home infusion pump, with the goal of eventually alerting the patients to correct mistakes before negative side effects could occur. Cameras are placed around a work table in the home dedicated to the medical activities. The system is trained on video recordings of other people operating the device correctly, and later detects the correct operation sequence in the currently performed procedure, and ultimately, provides corrective feedback to the user, including a portion of the recording that shows how the appropriate step should be performed. The videos are captured by stationary cameras and the direction of movement of the human activity is an important (non-invariant) vector to help recognize an action.

US 2009/0030286 A1 relates to a patient operable data collection system, where a patient places a probe on his body at a location specified by a local interface device including a display screen that displays a diagram showing the desired location for the patient to place the probe. The local interface may be implemented on a handheld device such as a cellular telephone or PDA. When the probe is utilized by the patient, the system will show the patient how to use the probes and record the output of the probes at the correct locations. If the probe's output differs significantly when placed at the incorrect locations in the vicinity of the correct locations, the differences can be used to direct the patient to move the probe to the correct location.

However, when a non-professional user makes an attempt on measurement at home, his or her measurement values may be abnormal due to various causes such as wrong measurement methods, failed measurement devices, and the like. Accordingly, the professionals cannot accurately determine such a situation only with measurement values transmitted from a health care server, thereby causing a problem in which the accuracy and reliability of measurement values are reduced.

### DISCLOSURE OF THE INVENTION

An aspect of the present disclosure is to provide a health care system and a health care service based on video in a remote health care solution.

In order to accomplish the foregoing objects, a method of providing a health care service through a terminal and a corresponding terminal, according to the claims, are provided.

An exemplary method for explaining the present disclosure may include acquiring an image in which a health care service user's health care behavior is captured and the measurement information of a measurement device associated with the health care behavior; determining whether or not the health care behavior is normally carried out based on the captured image or the measurement information of the measurement device; and displaying an indicator indicating that the health care behavior is not normally carried out on a screen of the terminal when the health care behavior is not normally carried out.

According to an example associated with the present disclosure, the health care behavior may be at least one of the user's blood glucose measuring behavior, the user's blood pressure measuring behavior, the user's eating behavior, the user's medication behavior and the user's exercise behavior.

According to an example associated with the present disclosure, the measurement information of the measurement device may be at least one of the user's blood glucose level, the user's blood pressure value, the user's medication dosage, the user's food intake amount, the user's workrate, the power status of the measurement device, the operation or non-operation of the measurement device, a humidity and temperature during measurement.

According to an example associated with the present disclosure, said determining whether or not the user's health care is normally carried out may be determined based on at least one of whether or not the subject of the health care behavior is a valid user of the health care service, the user's posture during measurement, whether or not the measurement device is used in a right manner, a failure or non-failure during the operation of the measurement device, and whether or not the measurement information is abnormal.

According to an example associated with the present disclosure, the foregoing method may further include displaying an image in which the health care behavior is captured on the screen of the terminal, wherein the indicator is displayed at a portion where the health care behavior is abnormally carried out on the displayed screen.

According to an example associated with the present disclosure, the captured image and the indicator may be displayed on the screen of the terminal while the health care behavior is carried out.

According to an example associated with the present disclosure, the foregoing method may further include providing guide information for carrying out a normal health care behavior again.

According to an example associated with the present disclosure, the provision of the guide information may be provided through at least one of an indicator, an image and a voice indicating the guide information.

According to an example associated with the present disclosure, the foregoing method may further include generating a control signal for controlling the measurement device to provide information indicating that the health care behavior is abnormally carried out to the user when the health care behavior is not normally carried out.

According to an example associated with the present disclosure, the information indicating that the health care behavior is abnormally carried out may be provided to the user through at least one of a vibration, an image and a voice output by means of the measurement device.

According to an example associated with the present disclosure, the foregoing method may further include transmitting at least one of the captured images and the measurement information of the measurement device to a health care server when the health care behavior is not normally carried out.

In order to accomplish the forgoing aspects, a terminal according to the present disclosure may include a display unit configured to display a screen; and a controller configured to acquire an image in which a health care service user's health care behavior is captured and the measurement information of a measurement device associated with the health care behavior, determine whether or not the health care behavior is normally carried out based on the captured image or the measurement information of the measurement device, and display an indicator indicating that the health care behavior is not normally carried out on a screen of the terminal when the health care behavior is not normally carried out.

According to an example associated with the present disclosure, the health care behavior may be at least one of the user's blood glucose measuring behavior, the user's blood pressure measuring behavior, the user's eating behavior, the user's medication behavior and the user's exercise behavior.

According to an example associated with the present disclosure, the measurement information of the measurement device may be at least one of the user's blood glucose level, the user's blood pressure value, the user's medication dosage, the user's food intake amount, the user's workrate, the power status of the measurement device, the operation or non-operation of the measurement device, a humidity and temperature during measurement.

According to an example associated with the present disclosure, the determination of whether or not the user's health care is normally carried out may be determined based on at least one of whether or not the subject of the health care behavior is a valid user of the health care service, the user's posture during measurement, whether or not the measurement device is used in a right manner, a failure or non-failure during the operation of the measurement device, and whether or not the measurement information is abnormal.

According to an example associated with the present disclosure, there is provided a health care service or system for determining whether or not the health care behavior is normally carried out based on an image in which the health care service user's health care behavior is captured and the measurement information of the measurement device associated with the health care behavior, and providing an indicator indicating that the health care behavior is not normally carried out or guide information to carry out a normal health care behavior when the health care behavior is not normally carried out.

In particular, according to a method of providing a health care service through a terminal disclosed in the present disclosure, a health care service user may receive an indicator indicating that the health care behavior is not normally carried out or guide information for carrying out a normal health care behavior when the health care behavior is not normally carried out while the user personally performs a health care behavior, thereby carrying out the health care behavior in an accurate and effective manner.

### BRIEF DESCRIPTION OF THE DRAWINGS

FIG. 1 is an exemplary view conceptually illustrating the structure of a health care system according to the present disclosure;
FIG. 2 is a block diagram illustrating the configuration of a mobile terminal 100 in the health care system;
FIG. 3 is a block diagram illustrating the configuration of a terminal;
FIG. 4 is a flow chart illustrating an exemplary method of providing a health care service through a terminal to understand the present disclosure;
FIG. 5 is an exemplary view illustrating an example of a method of providing a health care service through a terminal according to an embodiment disclosed in the present disclosure;
FIG. 6 is an exemplary view illustrating another example of a method of providing a health care service through a terminal according to the embodiment disclosed in the present disclosure;
FIG. 7 is an exemplary view illustrating an exemplary method of providing a health care service through a terminal to understand the present disclosure;
FIG. 8 is an exemplary view illustrating another exemplary method of providing a health care service through a terminal to understand the present disclosure;
FIG. 9 is an exemplary view illustrating an example of a method of displaying an indicator according to an embodiment disclosed in the present disclosure;
FIG. 10 is an exemplary view illustrating an example of a method of displaying guide information according to an embodiment disclosed in the present disclosure;
FIG. 11 is a flow chart illustrating a method of providing a health care service according to an embodiment disclosed in the present disclosure;
FIG. 12 is an exemplary view illustrating a method of providing a health care service to understand the present disclosure; and
FIG. 13 is a flow chart illustrating a method of providing a health care service to understand the present disclosure.

### DETAILED DESCRIPTION

Technologies disclosed in the present disclosure may be applicable to a health care system or a method of providing health care contents. However, technologies disclosed in the present disclosure may be also applicable to all health information providing systems and control methods thereof, mobile terminals (or portable devices) constituting the health information providing systems, health care servers and control methods thereof, and methods of collecting, processing and using health information transmitted in a wired and wireless manner, to which the technical concept of the technologies is applicable.

The health care system in particular relates to a health care service system and a method thereof using a ubiquitous network capable of continuously monitoring locations, health conditions and workrates for a plurality of users in a particular region, implementing the health care and exercise care of the plurality of users, preventing an emergency situation in advance based on the location information, health conditions and workrates, and quickly responding to the occurrence of an emergency situation.

It should be noted that technological terms used herein are merely used to describe a specific embodiment or examples, but not to limit the present invention. Also, unless particularly defined otherwise, technological terms used herein should be construed as a meaning that is generally understood by those having ordinary skill in the art to which the invention pertains, and should not be construed too broadly or too narrowly. Furthermore, if technological terms used herein are wrong terms unable to correctly express the concept of the invention, then they should be replaced by technological terms that are properly understood by those skilled in the art. In addition, general terms used in this invention should be construed based on the definition of dictionary, or the context, and should not be construed too broadly or too narrowly.

Incidentally, unless clearly used otherwise, expressions in the singular number include a plural meaning. In this application, the terms "comprising" and "including" should not be construed to necessarily include all of the elements or steps disclosed herein, and should be construed not to include some of the elements or steps thereof, or should be construed to further include additional elements or steps.

Furthermore, a suffix "module" or "unit" used for constituent elements disclosed in the following description is merely intended for easy description of the specification, and the suffix itself does not give any special meaning or function.

Furthermore, in the present disclosure, the terms including an ordinal number such as first, second, etc. can be used to describe various elements, but the elements should not be limited by those terms. The terms are used merely for the purpose to distinguish an element from the other element. For example, a first element may be named to a second element, and similarly, a second element may be named to a first element without departing from the scope of right of the invention.

Hereinafter, the embodiment and examples disclosed herein will be described in detail with reference to the accompanying drawings, and the same or similar elements are designated with the same numeral references regardless of the numerals in the drawings and their redundant description will be omitted.

In describing the present disclosure, moreover, the detailed description will be omitted when a specific description for publicly known technologies to which the invention pertains is judged to obscure the gist of the present invention. Also, it should be noted that the accompanying drawings are merely illustrated to easily explain the concept of the invention, and therefore, they should not be construed to limit the technological concept disclosed herein by the accompanying drawings.

In general, a health care system or health information providing system refers to a system for comprehensively providing a health care service by receiving various health care related information from a customer (ordinary person or patient) or providing various health care related information to the customer (ordinary person or patient). For example, the health care system may comprehensively support the search of various medical information, the promotion of customer's health, self-diagnostics, the scheduling of appointments, comparative evaluation information on various products, information required for the management of specialized medical institutions, and online transactions.

In recent years, with an increase of interest in health, studies on health care systems for more effective health care and health devices constituting those health care systems have been continuously carried out. Technologies for health devices capable of checking personal health, such as a fall sensor, a medication sensor, an electrocardiogram sensor and the like into which a blood pressure meter, a blood glucose meter, a body fat analyser, or the like and IT technologies are merged have been continually developed.

Owing to the individual development of such a health care system and a health device related thereto, technologies for comprehensively managing various health care systems and information on health devices related thereto are required, and thus there are provided ISO/IEEE 11073 Personal Health Device (hereinafter, referred to as a "PHD standard") as a standard related to personal health devices and HL7 as a medical information related international standard.

The PHD standard is a standard for providing interoperability between personal health devices, and the foregoing standard defines specialization for each personal health device upon the 11073-20601 based standards such that personal health devices are operated in a plug-and-play manner.

Communication protocols applicable to the PHD are as follows.

First, the local area communication protocol may include Bluetooth, RFID (Radio Frequency Identification), IrDA (infrared Data Association), UWB (Ultra Wideband), ZigBee, Wireless LAN (protocols such as Bluetooth, 802.11n, etc.), and the like.

In addition, the wireless Internet technology may include WLAN (Wireless LAN), Wi-Fi, WiBro (Wireless Broadband), WiMAX (World Interoperability for Microwave Access), HSDPA (High Speed Downlink Packet Access), and the like.

Furthermore, the wired communication related protocol or interface may include a USB (Universal Serial Bus) port, a HDMI (High-Definition Multimedia Interface) port, a DP (Display Port), a wired/wireless headset port, an external charger port, a wired/wireless data port, a memory card port, a port for connecting a device provided with an identification module, an audio I/O (Input/Output) port, a video I/O (Input/Output) port, an earphone port, and the like.

The HL7 standard provides a standard related to clinical and health data as an international standard mostly used in EHR (Electronic Health Record) systems in a hospital. The HL7 standard largely includes a V2 message and a V3 CDA (Clinical Document Architecture).

A health care system according to the present disclosure may be a total health care system for comprehensively providing health care, appointment scheduling, decision-making, consulting, and online transaction services to customers using a communication network.

Furthermore, a health care system according to the present disclosure may provide the comprehensive health care service through various applications to a customer. For example, the various applications may include at least one of an information search application, a health care application, a medical treatment application, a consulting application, and an electronic commerce application.

The information search application may perform the function of retrieving provider information including specialized medical information, drug information, disease information, hospitals, doctors, pharmacies, pharmaceutical companies and medical device manufacturers, medical related web sites, medical-related comparative evaluation information, and the like.

The health care application as an application of providing health care information for promoting a customer's disease prevention may perform the function of overall personal health care such as public health promotion, self-diagnostics, scheduling of appointments, health care after discharge, and the like. Furthermore, the health care application may perform the role of providing management of customer exercise information and exercise prescription, information on diet and prescription thereof, reservation for health care provides and counselling infrastructure, and health community and health contents. Furthermore, the health care application may perform a customized information service for frequently receiving customer's interested disease information.

The medical treatment application may perform the function of providing information on a patient to a healthcare professional when the healthcare professional takes care of the patient, thereby more effectively and accurately making his or her decision. For example, the information on a patient may be one of diagnosis information, treatment and prescription information.

The consulting information may provide legal, accounting, insurance and opening information required for medical facility management or information on areas of expertise associated with inventory management or the like as well as perform a consulting function through questions and answers with customers using on-line or off-line communication means.

The electronic commerce application supports typical on-line transactions, and may perform the function of managing a series of order, payment, and delivery processes for goods to be purchased by connecting at least one of the relevant shipping center, supplier and distributor when a customer (buyer) wants to purchase his or her desired medicines or medical device based on medical related comparative evaluation information.

### Description on the structure of health care system

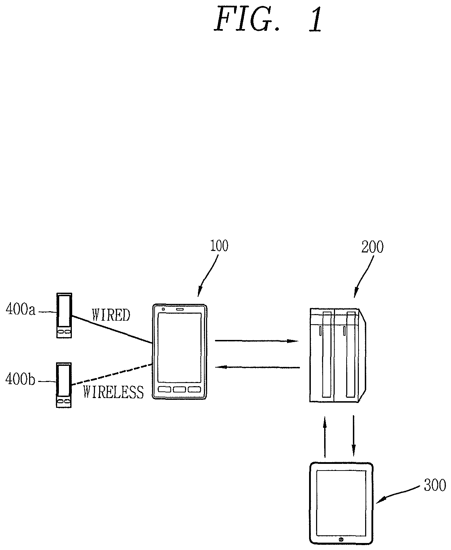

FIG. 1 is an exemplary view conceptually illustrating the structure of a health care system according to the present disclosure.

Referring to FIG. 1, a health care system may include a mobile terminal 100 and a health care server 200.

Furthermore, the health care system may further include a professional terminal 300 and a biometric measurement device 400. For example, a professional who uses the professional terminal 300 may include a doctor, a hospital, a pharmacist, a pharmacy, a medical consultant, a health care provider and a veterinarian. There may exist a plurality of biometric measurement devices 400, and the health care system in FIG. 1 may include a first biometric measurement device 400a and a second biometric measurement device 400b.

The constituent elements of the health care system as illustrated in FIG. 1 are not necessarily required, and the mobile terminal may be implemented with greater or less number of elements than those illustrated in FIG. 1.

Hereinafter, the constituent elements will be described in sequence.

The mobile terminal 100 may be functionally connected to at least one of the health care server 200, the professional terminal 300 and the biometric measurement device 400. To this end, the mobile terminal 100 may be connected to at least one of the health care server 200, the professional terminal 300 and the biometric measurement device 400 in a wired or wireless manner to exchange the health or biometric information of the customer (or user) with at least one of the health care server 200, the professional terminal 300 and the biometric measurement device 400.

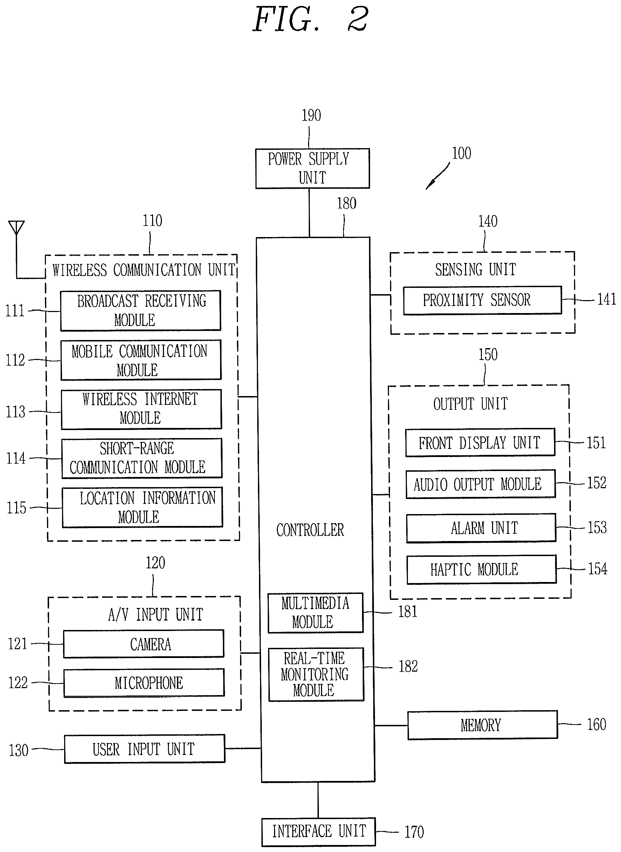

Furthermore, the customer (or user) of the health care system or service may carry the mobile terminal 100, and directly access information on various health care services such as health care, exercise management, medical examination, prescription, appointment, and the like. The mobile terminal 100 will be described later with respect to FIG. 2.

The health care server 200 may perform various functions for managing or controlling the entire health care system. For example, the health care server 200 may generate a survey or prescription related to health care, health status, exercise management or disease through the mobile terminal 100. Furthermore, the health care server 200 may receive information on the status, response of the customer for the survey or compliance with the prescription, and the like, and collect, generate, manage, store, provide or process the information, and transfer the result thereof again to the customer through the mobile terminal 100. To this end, the health care server 200 may include a central processing device (or processor, not shown).

The survey or prescription may be provided in the form of text or in the form of a web view through a web browser. To this end, the central processing device may transmit a script capable of generating the survey or prescription to the mobile terminal 100 or execute the script to transmit the execution result to the mobile terminal 100.

In this case, the mobile terminal 100 may show the script associated with the survey or prescription or the execution result of the script to the customer in the form of text or in a web view manner.

Furthermore, the health care server 200 may be functionally connected to at least one of the mobile terminal 100, the professional terminal 300 and the biometric measurement device 400, and to this end, connected to at least one of the mobile terminal 100, the professional terminal 300 and the biometric measurement device 400 through a wired or wireless communication network. To this end, the health care server 200 may include one or more communication modules (not shown).

Furthermore, the health care server 200 may collect various biometric information on the health care (or remote medical diagnosis) of a customer, information on customer's situation or information on customer's health status, and make database for the various information. To this end, the health care server 200 may be connected to an additional server having a storage medium capable of storing the database in a wired or wireless manner, and may further include a storage unit (not shown) therein.

The professional terminal 300 may be functionally connected to at least one of the health care server 200, the mobile terminal 100 and the biometric measurement device 400, and to this end, connected to at least one of the health care server 200, the mobile terminal 100 and the biometric measurement device 400 through a wired or wireless communication network.

The professional terminal 300 may perform various functions for at least one of customer's remote health care, remote exercise management and remote diagnosis. For example, the professional terminal 300 may transmit and/or receive the customer's health, exercise and medical information to and/or from the health care server 200, and through this, professionals including a doctor, a pharmacist or the like using the professional terminal 300 may remotely perform the customer's health care, exercise management or medical diagnosis.

The biometric measurement device 400 may perform the role of collecting various biometric information required for customer's health care or remote medical diagnosis. To this end, the biometric measurement device 400 may include various sensors and measurement devices for measuring customer's biometric information. For example, the biometric measurement device 400 may include at least one of a blood pressure meter, a blood glucose meter, a body fat analyser, an electrocardiogram sensor and the like. Furthermore, for example, the biometric measurement device 400 may include a fall sensor, a medication sensor, or other sensors capable of checking the customer's status into which IT technologies are merged.

Furthermore, the biometric measurement device 400 may include a sensor capable of measuring the user's workrate and caloric consumption based on the workrate or the like. The workrate may be calculated by acceleration, a number of steps, and the like. In addition, it should be understood by those skilled in the art that sensors capable of measuring various customer's biometric information, customer's condition or customer's status are provided in the biometric measurement device 400.

There are an electrocardiogram (ECG) sensor, a photoplethysmography (PPG) sensor and the like as a sensor for extracting information associated with heartbeat. In particular, PPG is a method for extracting information associated with heartbeat using a predetermined number of LEDs and optical detectors. Heartbeat detection using the PPG may be measured through only one contact point with a body using a simple sensor module, and thus more conveniently used compared to a method of using electrocardiogram or ECG in which two or more electrodes should be adhered.

The customer's biometric information may be the biometric information of a chronically ill patient, for example, information on pulse rate in case of a hypertensive patient, information on blood sugar level on in case of a diabetic patient, and information on electrocardiogram and pulse rate in case of a heart disease patient. Furthermore, the customer's biometric information may be a customer's workrate, a customer's body fat index, a customer's caloric value for digested food, a customer's biorhythm, and the like. In addition, it should be understood by those skilled in the art that various biometric information required for customer's health care or remote diagnosis are applicable to a health care system disclosed in the present disclosure.

Furthermore, the biometric measurement device 400 may collect context information on various types of customers for directly or indirectly showing a customer's body situation. For example, situation information on the customer, as information for stipulating the features of a situation such as a person, a place, a thing, an object, a time and the like that exerts an effect on interactions between a user and another user, system or device applications in association with ubiquitous computing, may include a computing context such as network connectivity, communication bandwidth, printer, display, workstation and the like, a user context such as user's profile, location, people around and the like, a physical context such as lighting, noise level, traffic condition, temperature and the like, and a time context such as time, week, month, season and the like.

According to a method of implementing the health care system, the biometric measurement device 400 may be provided in the mobile terminal 100 to measure a customer's biometric information. In this case, the mobile terminal 100 may additionally have a biometric module.

When the biometric measurement device 400 is separately formed from the mobile terminal 100, the biometric measurement device 400 may be connected to the mobile terminal 100 or the health care server 200 in a wired or wireless manner. For example, the biometric measurement device 400 may form a Bluetooth network through an access point (AP) to be connected to the mobile terminal 100 or the health care server 200 in a wireless manner.

In this case, the biometric measurement device 400 measures biometric information (for example, blood glucose, button portion, body composition, etc.) on a customer (or user) subject to health management, and transmits the biometric information to the mobile terminal 100 or the health care server 200 through the access point (AP) based on a wireless communication scheme.

In case of a plurality of customers (or users), the biometric measurement device 400 may identify the user through user identification (ID) information, and biometric information for each of the user identification (ID) information. In other words, when the measured biometric information is body component information, self identification (ID) information may be incorporated into the biometric measurement device 400 to identify a user so as to distinguish the user for each ID when ID is set during the measurement of biometric information, and the identification (ID) information may be used to distinguish the user during data management even in the health care server 200.

### Description of mobile terminal in health care system

FIG. 2 is a block diagram illustrating the configuration of a mobile terminal 100 in the health care system. The mobile terminal 100 may be implemented in various forms. For example, the mobile terminal (mobile phone) 100 may include a portable terminal, a smart phone, a laptop computer, a digital broadcast terminal, a personal digital assistant (PDA), a portable multimedia player (PMP), and the like.

As illustrated in FIG. 2, the mobile terminal 100 may include a wireless communication unit 110, an Audio/Video (A/V) input unit 120, a user input unit 130, a sensing unit 140, an output unit 150, a memory 160, an interface unit 170, a controller 180, a power supply unit 190, and the like. However, the constituent elements of the mobile terminal 100 as illustrated in FIG. 2 are not necessarily required, and the mobile terminal 100 may be implemented with greater or less number of elements than those elements illustrated in FIG. 2.

The wireless communication unit 110 typically includes one or more modules allowing radio communication between the mobile terminal 100 and a wireless communication system, or allowing radio communication between radio communication the mobile terminal 100 and a network in which the mobile terminal 100 is located. For example, the wireless communication unit 110 may include a broadcast receiving module 111, a mobile communication module 112, a wireless Internet module 113, a short-range communication module 114, a location information module 115, and the like.

As described above, the mobile terminal 100 may be functionally connected to at least one of the health care server 200, the professional terminal 300 and the biometric measurement device 400. To this end, the mobile terminal 100 may be connected to at least one of the health care server 200, the professional terminal 300 and the biometric measurement device 400 through the wireless communication unit 110 to exchange the health or biometric information of the customer (or user) with at least one of the health care server 200, the professional terminal 300 and the biometric measurement device 400.

The broadcast receiving module 111 receives broadcast signals and/or broadcast associated information from an external broadcast management server through a broadcast channel. The broadcast channel may include a satellite channel and/or a terrestrial channel. The broadcast management server may mean a server that generates and transmits a broadcast signal and/or broadcast associated information or a server that receives a previously generated broadcast signal and/or broadcast associated information and transmits to the mobile terminal 100. The broadcast associated information may mean information regarding a broadcast channel, a broadcast program, a broadcast service provider, and the like. The broadcast signal may include a TV broadcast signal, a radio broadcast signal and a data broadcast signal as well as a broadcast signal in a form that a data broadcast signal is combined with the TV or radio broadcast signal.

On the other hand, the broadcast associated information may also be provided through a mobile communication network, and in this case, the broadcast associated information may be received by the mobile communication module 112. The broadcast associated information may exist in various forms. For example, it may exist in the form of an electronic program guide (EPG) of digital multimedia broadcasting (DMB), electronic service guide (ESG) of digital video broadcast-handheld (DVB-H), and the like.

The broadcast receiving module 111 may receive a broadcast signal using various types of broadcast systems. In particular, the broadcast receiving module 111 may receive a digital broadcast signal using a digital broadcast system such as digital multimedia broadcasting-terrestrial (DMB-T), digital multimedia broadcasting-satellite (DMB-S), media forward link only (MediaFLO), digital video broadcast-handheld (DVB-H), integrated services digital broadcast-terrestrial (ISDB-T), and the like. The broadcast receiving module 111 is, of course, configured to be suitable for every broadcast system that provides a broadcast signal as well as the above-mentioned digital broadcast systems. The broadcast signal and/or broadcast-associated information received through the broadcast receiving module 111 may be stored in the memory 160.

The mobile communication module 112 transmits and/or receives a radio signal to and/or from at least one of a base station, an external terminal and a server over a mobile communication network. Here, the radio signal may include a voice call signal, a video call signal and/or various types of data according to text and/or multimedia message transmission and/or reception.

The wireless Internet module 113 means a module for supporting wireless Internet access. The wireless Internet module 113 may be built-in or externally installed to the mobile terminal 100. Here, it may be used a wireless Internet access technique including a WLAN (Wireless LAN), Wi-Fi, WiBro (Wireless Broadband), WiMAX (World Interoperability for Microwave Access), HSDPA (High Speed Downlink Packet Access), and the like.

The short-range communication module 114 is a module for supporting a short-range communication. Here, it may be used a short-range communication technology including Bluetooth, Radio Frequency IDentification (RFID), Infrared Data Association (IrDA), Ultra WideBand (UWB), ZigBee, Wireless LAN (protocols such as Bluetooth, 802.11n, etc.) and the like.

The location information module 115 is a module for checking or acquiring a location of the mobile terminal. A GPS module is an example. The GPS module receives location information from a plurality of satellites. Here, the location information may include coordinate information represented by latitude and longitude values. For example, the GPS module may measure an accurate time and distance from three or more satellites, and accurately calculate a current location according to trigonometry based upon three different distances. A method of acquiring distance and time information from three satellites and performing error correction with a single satellite may be used. In particular, the GPS module may acquire an accurate time together with three-dimensional speed information as well as the location of the latitude, longitude and altitude values from the location information received from the satellites. For the location information module 115, a Wi-Fi positioning system and/or a hybrid positioning system may be applicable thereto.

The A/V(audio/video) input unit 120 receives an audio or video signal, and the A/V (audio/video) input unit 120 may include a camera 121 and a microphone 122. The camera 121 processes an image frame, such as still picture or video, obtained by an image sensor in a video phone call or image capturing mode. The processed image frame may be displayed on a display unit 151.

The image frames processed by the camera 121 may be stored in the memory 160 (or other storage medium) or transmitted through the wireless communication unit 110. Two or more cameras 121 may be provided according to the configuration type and/or use environment of the mobile terminal.

The camera 121 may be used to check or monitor a customer's (or user's) health status or customer's current situation in a health care system. For example, the camera 121 may continuously capture the customer to monitor the customer's health status and workrate, thereby applying them to the customer's health care or exercise management. Furthermore, the 100 may analyze the capture result of the camera 121 to determine whether or not the customer's status is a dangerous situation, and transfer information on the situation to the health care server 200 connected thereto in a wired or wireless manner when in a dangerous situation. In this case, the health care server 200 may analyze and process information on the situation to transfer emergency treatment or action instructions for the situation to the customer through the mobile terminal 100. Through this, the health care system may provide services capable of preventing an emergency situation for the customer in advance, as well as quickly coping with the occurrence of an emergency situation.

The microphone 122 receives an external audio signal through a microphone in a phone call mode, a recording mode, a voice recognition mode, and the like, and processes the audio signal into electrical voice data. The processed voice data may be converted and outputted into a format capable of being transmitted to a mobile communication base station through the mobile communication module 112 in the phone call mode. The microphone 122 may implement various types of noise canceling algorithms to cancel noise generated in a procedure of receiving the external audio signal.

The user input unit 130 may generate input data to control an operation of the mobile terminal. The user input unit 130 may be configured by including a keypad, a dome switch, a touch pad (pressure/capacitance), a jog wheel, a jog switch, and the like. Particularly, when the touch pad forms an interlayer structure together with a display unit 151, it may be called a touch screen.

The sensing unit 140 detects a current status of the mobile communication terminal 100 such as an opened or closed state of the mobile communication terminal 100, a location of the mobile communication terminal 100, the presence or absence of user contact, an orientation of the mobile communication terminal 100, an acceleration or deceleration movement of the mobile communication terminal 100, and the like, and generates a sensing signal for controlling the operation of the mobile communication terminal 100. For example, when the mobile communication terminal 100 is a slide phone type, it may sense an opened or closed state of the slide phone. Furthermore, the sensing unit 140 takes charge of a sensing function associated with whether or not power is supplied from the power supply unit 190, whether or not an external device is coupled with the interface unit 170.

Furthermore, the sensing unit 140 may measure a customer's biometric information using a health care system. The customer's biometric information may be the biometric information of a chronically ill patient, for example, information on pulse rate in case of a hypertensive patient, information on blood sugar level on in case of a diabetic patient, and information on electrocardiogram and pulse rate in case of a heart disease patient. Furthermore, the customer's biometric information may be a customer's workrate, a customer's body fat index, a customer's caloric value for digested food, a customer's biorhythm, and the like. In addition, it should be understood by those skilled in the art that various biometric information required for customer's health care or remote diagnosis are applicable to a health care system disclosed in the present disclosure.

The sensing unit 140 may further include a biometric information measurement sensor 142 to measure the biometric information. As described above, the mobile terminal 100 may be connected to the biometric measurement device 400 physically separated therefrom in a wired or wireless manner to receive the biometric information, but the biometric information measurement sensor 142 may be additionally provided in the sensing unit 140 to collect the biometric information.

Furthermore, the biometric information measurement sensor 142 may collect context information on various types of customers for directly or indirectly showing a customer's body situation. For example, situation information on the customer, as information for stipulating the features of a situation such as a person, a place, a thing, an object, a time and the like that exerts an effect on interactions between a user and another user, system or device applications in association with ubiquitous computing, may include a computing context such as network connectivity, communication bandwidth, printer, display, workstation and the like, a user context such as user's profile, location, people around and the like, a physical context such as lighting, noise level, traffic condition, temperature and the like, and a time context such as time, week, month, season and the like.

The interface unit 170 may perform the role of interfacing with all external devices connected to the mobile terminal 100. The interface unit 170 may include, for example, a universal serial bus (USB) port, a high-definition multimedia interface (HDMI) port, a display ports (DP), a wired/wireless headset port, an external charger port, a wired/wireless data port, a memory card port, a port for coupling devices having an identification module, audio Input/Output (I/O) ports, video I/O ports, earphone ports, and the like. Here, the identification module may be configured as a chip for storing various information required to authenticate an authority to use the mobile terminal 100, which may include a User Identity Module (UIM), a Subscriber Identity Module (SIM), a Universal Subscriber Identity Module (USIM) and the like. Also, the device having the identification module (hereinafter, referred to as "identification device") may be implemented in a type of smart card. Hence, the identification device can be coupled to the mobile terminal 100 via a port. The interface unit 170 may receive data or power from an external device to transfer it to each constituent element in the mobile terminal 100 or transmit data within the mobile terminal 100 to the external device.

As described above, the mobile terminal 100 may be functionally connected to at least one of the health care server 200, the professional terminal 300 and the biometric measurement device 400. To this end, the mobile terminal 100 may be connected to at least one of the health care server 200, the professional terminal 300 and the biometric measurement device 400 through the interface unit 170 in a wired manner to exchange the health or biometric information of the customer (or user) with at least one of the health care server 200, the professional terminal 300 and the biometric measurement device 400.

The output unit 150 is configured to provide an output for audio signal, video signal, or alarm signal, and the output unit 150 may include the display unit 151, an audio output module 152, an alarm unit 153, and the like.

The display unit 151 may display (output) information processed in the mobile terminal 100. For example, when the mobile terminal 100 is in a phone call mode, the display unit 151 may display a User Interface (UI) or a Graphic User Interface (GUI) associated with a call. When the mobile terminal 100 is in a video call mode or image capture mode, the display unit 151 may display a captured image and/or received image, a UI or GUI.

The display unit 151 may include at least one of a liquid crystal display, a thin film transistor-liquid crystal display, an organic light emitting diode display, a flexible display, an a three-dimensional (3D) display. Two or more display units 151 may be implemented according to a configured aspect of the mobile terminal 100. For instance, both an external display unit (not shown) and an internal display unit (not shown) may be provided in the mobile terminal 100.

When the display unit 151 and a touch sensitive sensor (hereinafter, referred to as a "touch sensor") have an interlayer structure (hereinafter, referred to as a "touch screen"), the display unit 151 may be used as an input device rather than an output device. The touch sensor may be implemented as a touch film, a touch sheet, a touch pad, and the like.

Furthermore, the touch sensor may be configured to convert changes of a pressure applied to a specific part of the display unit 151, or a capacitance occurring from a specific part of the display unit 151, into electric input signals. Also, the touch sensor may be configured to sense not only a touched position and a touched area, but also a touch pressure. When there is a touch input to the touch sensor, the corresponding signals are transmitted to a touch controller (not shown). The touch controller processes the received signals, and then transmits corresponding data to the controller 180. Accordingly, the controller 180 may sense which region of the display unit 151 has been touched. Furthermore, the controller 180 can perform a pattern recognition processing so as to recognize writing or drawing input carried out on the touch screen as text or image.

The proximity sensor 141 may be arranged at an inner region of the mobile terminal 100 covered by the touch screen, or near the touch screen. The proximity sensor 141 indicates a sensor to sense presence or absence of an object approaching to a surface to be sensed, or an object disposed near a surface to be sensed, by using an electromagnetic field or infrared rays without a mechanical contact. The proximity sensor has a longer lifespan and a more enhanced utility than a contact sensor.

The proximity sensor 141 may include an optical transmission type photoelectric sensor, a direct reflective type photoelectric sensor, a mirror reflective type photoelectric sensor, a high-frequency oscillation proximity sensor, a capacitance type proximity sensor, a magnetic type proximity sensor, an infrared rays proximity sensor, and so on. When the touch screen is implemented as a capacitance type, proximity of a pointer to the touch screen is sensed by changes of an electromagnetic field. In this case, the touch screen (touch sensor) may be categorized into a proximity sensor.

Hereinafter, for the sake of convenience of brief explanation, a status that the pointer is positioned to be proximate onto the touch screen without contact will be referred to as "proximity touch", whereas a status that the pointer substantially comes in contact with the touch screen will be referred to as "contact touch". For the position corresponding to the proximity touch of the pointer on the touch screen, such position corresponds to a position where the pointer faces perpendicular to the touch screen upon the proximity touch of the pointer.

Furthermore, the proximity sensor 141 senses proximity touch, and proximity touch patterns (e.g., distance, direction, speed, time, position, moving status, etc.). Information relating to the sensed proximity touch and the sensed proximity touch patterns may be output onto the touch screen.

The audio output module 152 may output audio data received from the wireless communication unit 110 or stored in the memory 160, in a call-receiving mode, a call-placing mode, a recording mode, a voice recognition mode, a broadcast reception mode, and so on. The audio output module 152 may output audio signals relating to functions performed in the mobile terminal 100, e.g., sound alarming a call received or a message received, and so on. The audio output module 152 may include a receiver, a speaker, a buzzer, and so on.

The alarm 153 outputs signals notifying occurrence of events from the portable terminal 100. The events occurring from the mobile terminal may include call received, message received, key signal input, touch input, and so on. The alarm 153 may output not only video or audio signals, but also other types of signals such as signals notifying occurrence of events in a vibration manner. When a call signal or message is received, the alarm 153 may output a vibration to notify this. Otherwise, when a key signal is inputted, the alarm 153 may output a vibration as a feedback to the inputted key signal. Through the foregoing vibration output, the user can recognize an event occurrence. The signal for notifying an event occurrence may be also outputted through the display unit 151 or the audio output module 152.

The haptic module 154 generates various tactile effects which a user can feel. A representative example of the tactile effects generated by the haptic module 154 includes vibration. Vibration generated by the haptic module 154 may have a controllable intensity, a controllable pattern, and so on. For instance, different vibration may be output in a synthesized manner or in a sequential manner.

The haptic module 154 may generate various tactile effects, including not only vibration, but also arrangement of pins vertically moving with respect to a skin being touched, air injection force or air suction force through an injection hole or a suction hole, touch by a skin surface, presence or absence of contact with an electrode, effects by stimulus such as an electrostatic force, reproduction of cold or hot feeling using a heat absorbing device or a heat emitting device, and the like.

The haptic module 154 may be configured to transmit tactile effects through a user's direct contact, or a user's muscular sense using a finger or a hand. The haptic module 154 may be implemented in two or more in number according to the configuration of the mobile terminal 100. For example, it may be provided in a steering wheel, a gearshift lever, a seat, and the like.

The memory 160 may store a program for processing and controlling the controller 180. Alternatively, the memory 160 may temporarily store input/output data (e.g., a customer's unique ID, etc.).

Furthermore, the memory 160 may store a customer's real-time monitoring results by means of the mobile terminal 100 such as the customer's health status, workrate check or status of compliance with a prescription for disease, and the like.

Furthermore, the memory 160 may store customer information (for example, user's measurement history, etc.) analyzed and processed by the controller 180 with a database.

Furthermore, the memory 160 may store a customer's biometric information measured through the biometric information measurement sensor 142, and the customer's image or picture captured through the camera 121.

Also, the memory 160 may store data related to various patterns of vibrations and sounds outputted upon the touch input on the touch screen. The memory 160 may be implemented using any type of suitable storage medium including a flash memory type, a hard disk type, a multimedia card micro type, a memory card type (e.g., SD or DX memory), Random Access Memory (RAM), Static Random Access Memory (SRAM), Read-Only Memory (ROM), Electrically Erasable Programmable Read-only Memory (EEPROM), Programmable Read-only Memory (PROM), magnetic memory, magnetic disk, optical disk, and the like. Also, the mobile terminal 100 may operate a web storage which performs the storage function of the memory 160 on the Internet.

The interface unit 170 may generally be implemented to interface the mobile device with external devices. The interface unit 170 may allow a data reception from an external device, a power delivery to each component in the mobile terminal 100, or a data transmission from the mobile terminal 100 to an external device. The interface unit 170 may include, for example, wired/wireless headset ports, external charger ports, wired/wireless data ports, memory card ports, ports for coupling devices having an identification module, audio Input/Output (I/O) ports, video I/O ports, earphone ports, and the like. Here, the identification module may be configured as a chip for storing various information required to authenticate an authority to use the mobile terminal 100, which may include a User Identity Module (UIM), a Subscriber Identity Module (SIM), and the like. Also, the device having the identification module (hereinafter, referred to as "identification device") may be implemented in a type of smart card. Hence, the identification device can be coupled to the mobile terminal 100 via a port. The interface unit 170 may receive data or power from an external device to transfer it to each constituent element in the mobile terminal 100 or transmit data within the mobile terminal 100 to the external device.

The interface unit 170 may serve as a path for power to be supplied from an external cradle to the mobile terminal 100 when the mobile terminal 100 is connected to the external cradle or as a path for transferring various command signals inputted from the cradle by a user to the mobile terminal 100. Such various command signals or power inputted from the cradle may operate as signals for recognizing that the mobile device has accurately been mounted to the cradle.

The controller 180 typically controls the overall operations of the mobile terminal 100 for providing a health care service to a customer. For example, the controller 180 may perform the control and processing in association with collecting the customer's biometric information, displaying a survey or prescription generated from the health care server 200 on the screen, and transferring the customer's response to this to the health care server 200.

The controller 180 may include an analysis module 181. Furthermore, the controller 180 may further include a real-time monitoring module 182. All the constituent elements may not be necessarily required to provide a health care system or server, and the controller 180 may be implemented with greater or less number of elements than those illustrated in the above.

The analysis module 181 may perform the role of analyzing various information on a customer. For example, the analysis module 181 may analyze, process and handle the measured customer's biometric information, and store the results with a database in the memory 160.

Furthermore, the analysis module 181 may transfer the customer's biometric information to the health care server 200, thereby allowing the health care server 200 to perform diagnosis for the customer's health care, exercise management and disease or treatment for disease, and the like.

Furthermore, the analysis module 181 may determine whether or not there is an error for various biometric information on the measured customer, and control the biometric information measurement sensor to make a re-measurement when there is an error as a result of the determination.

The real-time monitoring module 182 may perform the role of controlling the camera 121 or the biometric information measurement sensor 142 to monitor the customer in a real-time manner to check the customer's health status, customer's workrate or compliance with a specialist's prescription in a health care system.

Furthermore, the real-time monitoring module 182 may store the real-time monitoring results with a database in the memory 160.

Furthermore, the real-time monitoring module 182 may transfer the real-time monitoring results to the health care server 200, and thus the health care server 200 may analyze, process and handle the real-time monitoring results to perform diagnosis or treatment according to the results.

The power supply unit 190 receives external power and internal power under the control of the controller 180 to provide power required by various components.

The function of a constituent element applied to the mobile terminal 100 may be implemented in a medium that can be read by a computer or similar device using software, hardware, or any combination thereof. For hardware implementation, it may be implemented by using at least one of application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, electrical units designed to perform the functions described herein. In some cases, it may be implemented in the controller 180 itself. For software implementation, procedures or functions may be implemented together with separate software modules. The software modules may perform at least one function or operation described herein. Software codes can be implemented by a software application written in any suitable programming language. The software codes may be stored in the memory 160 and executed by the controller 180.

### Description on the configuration of terminal

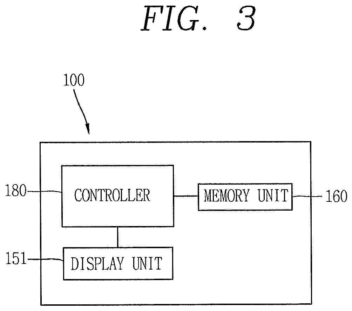

FIG. 3 is a block diagram illustrating the configuration of a terminal to understand the present disclosure.

Referring to FIG. 3, the terminal 100 may include the controller 180. Furthermore, the terminal 100 may further include a memory unit 160 and a display unit 151. In addition, the terminal 100 may further include various constituent elements to perform a health care service for providing a self-aware reminder for health care motivation.

The constituent elements of the terminal 100 as illustrated in FIG. 3 are not necessarily required, and the mobile terminal may be implemented with greater or less number of elements than those illustrated in FIG. 1.

Here, the terminal in FIG. 3 may be configured in a stand-alone manner as well as applicable to various terminals such as a mobile terminal, a telematics terminal, a smart phone, a portable terminal, a personal digital assistant (PDA), a portable multimedia player (PMP), a desktop pc, a laptop computer, a tablet PC), WiBro terminal, Internet Protocol Television (IPTV) terminal, a television set, 3D television set, an image device, a navigation terminal, an audio video navigation (AVN) terminal, and the like.

Hereinafter, the constituent elements will be described in sequence.

The controller 180 may control various constituent elements constituting the terminal 100 to carry out a more accurate and effective user health care behavior through the accuracy determination of a video based user health care behavior.

The controller 180 may acquire an image in which the health care service user's health care behavior has been captured and the measurement information of a measurement device associated with the health care behavior.

The health care behavior may be at least one of the user's blood glucose measuring behavior, the user's blood pressure measuring behavior, the user's eating behavior, the user's medication behavior and the user's exercise behavior.

For example, the captured image may be an image that has captured a scene in which the user measures his or her blood pressure when the user measures his or her blood pressure through a blood pressure measurement device.

The controller 180 may acquire the captured image through a camera 121 provided in the terminal 100. However, the terminal 100 may acquire the image through an external device connected through the interface unit 170 of the terminal 100. Otherwise, the controller 180 may acquire the image that has been prestored from an external server connected thereto in a wired or wireless manner. In addition, it should be understood by those skilled in the art that various image acquisition methods can be applicable to a health care service method through a terminal disclosed in the present disclosure.

Here, measurement as quantitatively capturing a fact (for example, user's blood pressure or blood glucose) using various methods and devices may imply broader content than the measurement. For example, the measurement may be measuring the user's food intake amount or the user's workrate.

A measurement device associated with the health care behavior may denote an apparatus or device capable of acquiring various health information associated with the health care behavior. For example, when the user's health care behavior is an eating behavior, the measurement device may measure the user's food intake amount. For example, when the health care behavior is the user's medication behavior, the measurement device may be a measurement device mounted on a medication container to measure capable of measuring the medication dosage of the medicine.

Furthermore, the measurement device may be a meter for measuring the user's biometric information (for example, user's blood glucose, blood pressure, heart rate etc.). For example, the measurement device may be the foregoing biometric measurement device 400. In other words, the controller 180 may acquire the user's biometric information measured from the biometric measurement device 400 connected thereto in a wired or wireless manner.

Furthermore, for example, the measurement device may be a biometric information measurement sensor 142 provided in the terminal 100. Accordingly, the controller 180 may acquire the user's biometric information measured from the biometric information measurement sensor.

The measurement information of the measurement device is at least one of the user's blood glucose level, the user's blood pressure value, the user's medication dosage, the user's food intake amount, the user's workrate, the power status of the measurement device, the operation or non-operation of the measurement device, a humidity and temperature during measurement.

The controller 180 may determined whether or not the health care behavior is normally carried out based on the captured image or the measurement information of the measurement device.

The determination of whether or not the user's health care is normally carried out may be determined based on at least one of whether or not the subject of the health care behavior is a valid user of the health care service, the user's posture during measurement, whether or not the measurement device is used in a right manner, a failure or non-failure during the operation of the measurement device, and whether or not the measurement information is abnormal.

For example, when the measured user's blood pressure value is a value that cannot be measured in a typical measurement condition, the controller 180 may determine that the measurement is not normally carried out. For example, furthermore, when the measurement position of the blood pressure measurement device is wrong based on the analysis result of the captured image in case where the health care behavior is the user's blood pressure measurement behavior, the controller 180 may determined that the health care behavior is not normally carried out.

Furthermore, the controller 180 may display an indicator indicating that the health care behavior is not normally carried out when the health care behavior is not normally carried out.

The indicator may be expressed in various forms to indicate that the health care behavior is not normally carried out. For example, the indicator may be a character indicating a professional (doctor, a pharmacist or health care provider, etc.). Furthermore, for example, the indicator may be a symbol (for example, exclamation mark) indicating a warning. In addition, it should be understood by those skilled in the art that various forms of indicators can be provided in the method of providing a health care service disclosed in the present disclosure.

The memory unit 160 may store the customer's real-time monitoring results by means of the mobile terminal 100 such as the user's health status, workrate check or status of compliance with a prescription for disease, and the like.

Furthermore, the memory 160 may store customer information (for example, user's measurement history, etc.) analyzed and processed by the controller 180 with a database.

The memory unit 160 may store an image in which the user's health care behavior is captured and the may include of the measurement device.

Furthermore, the memory unit 160 may store the result of analyzing, processing, and handling the captured image or the measurement information.

The display unit 151 may display an image associated with a health care service. In other words, the display unit 151 may display and output information processed by the terminal 100. For example, the terminal 100 may display a user interface (UI) or graphical user interface (GUI) for providing the health care service to a user (or customer).

The display unit 151 may display an imaged in which the health care behavior is captured on the screen of the terminal 100. It may be aimed for the user to confirm his or her own behavior through the terminal 100 and check whether or not the health care behavior is normally carried out.

Furthermore, the display unit 151 may display the indicator at a portion where the health care behavior is abnormally carried out on the displayed screen. Through this, the user may know at which portion the health care behavior is wrong.

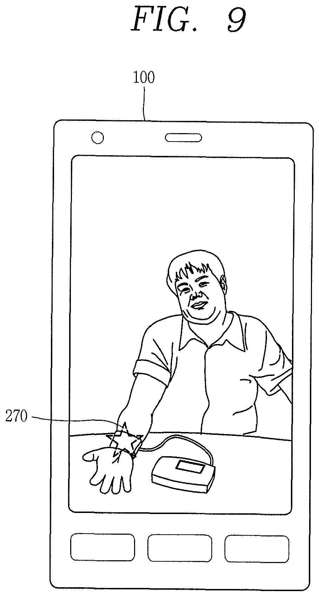

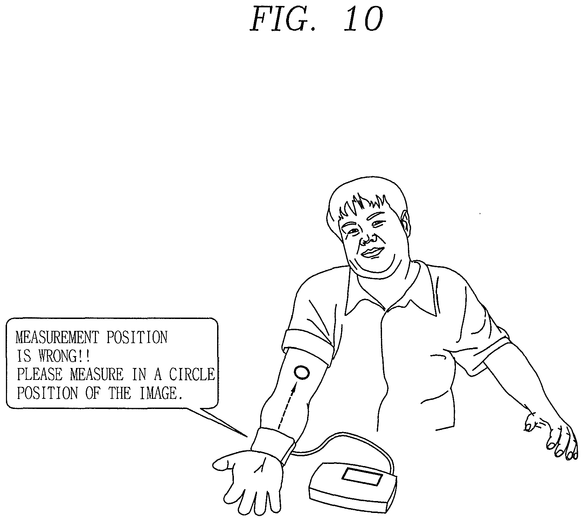

Furthermore, the captured image and the indicator may be displayed on the screen of the terminal while carrying out the health care behavior. Through this, the user may check his or her own behavior in real time while performing the health care behavior. Furthermore, as a result of the check, when there is a wrong behavior or posture, the user may immediately modify the health care behavior to have a chance for a retry. It will be described below in detail with reference to FIGS. 9 and 10.

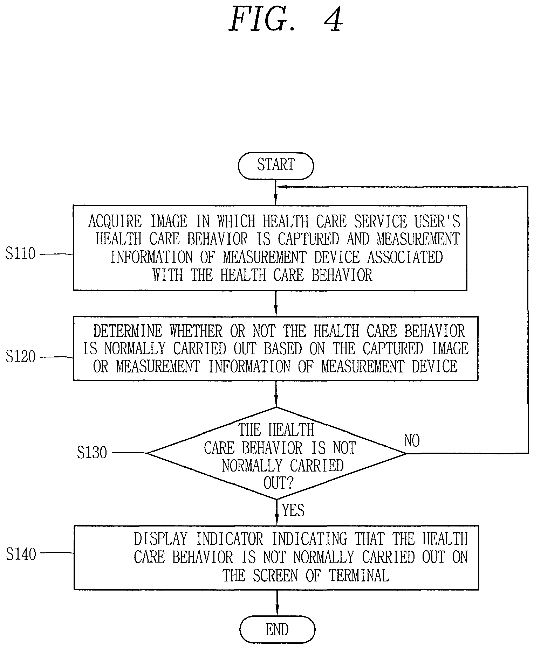

Hereinafter, an exemplary method of providing a health care service through a terminal to understand the present disclosure will be described below with reference to FIG. 4.

### Description on method of providing health care service through terminal

FIG. 4 is a flow chart illustrating an exemplary method of providing a health care service through a terminal to understand the present disclosure.

Referring to FIG. 4, an exemplary method of providing a health care service through a terminal may be carried out with the following steps.

First, the terminal 100 may acquire an image in which a health care service user's health care behavior is captured and the measurement information of a measurement device associated with the health care behavior (S110).

Next, the terminal 100 may determine whether or not the health care behavior is normally carried out based on the captured image or the measurement information of the measurement device (S120).

Next, when the health care behavior is not normally carried out (S130), the terminal 100 may display an indicator indicating that the health care behavior is not normally carried out on the screen of the terminal (S140).

The terminal 100 may have various criteria for the health care behavior to determine whether or not the health care behavior is normally carried out. For example, the criteria may be at least one of a correct posture for the health care behavior, a normal operating condition of the measurement device, a measurement associated with the health care behavior, and a temperature and humidity for normally performing a measurement associated with the health care behavior.

The determination criteria may be acquired using various methods. For example, the terminal 100 may acquire the criteria from the health care server 200. For example, furthermore, the terminal 100 may receive information on the criteria from a user. In addition, it should be understood by those skilled in the art that various methods for acquiring the criteria could be applicable to a method of providing a health care service disclosed in the present disclosure.

Furthermore, the terminal 100 may store the acquired criteria in the memory unit 160. For example, the terminal 100 may acquire the captured image and measurement information on the measurement device, and then compare them with the criteria prestored in the memory unit 160 to determine whether or not the health care behavior is normally carried out.

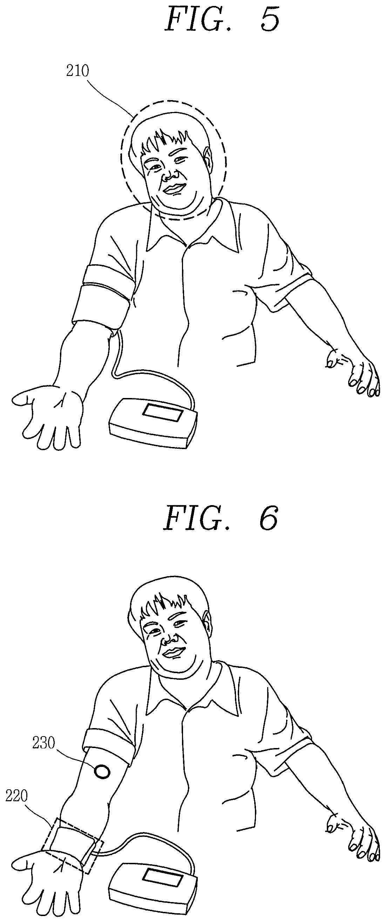

Hereinafter, a method of determining whether or not the health care behavior is normally carried out will be described with reference to FIGS. 5 through 8.

According to an embodiment disclosed in the present disclosure, during the determination of whether or not the user's health care is normally carried out, the controller 180 may determine it based on at least one of whether or not the subject of the health care behavior is a valid user of the health care service, the user's posture during measurement, whether or not the measurement device is used in a right manner, a failure or non-failure during the operation of the measurement device, and whether or not the measurement information is abnormal.

To this end, the controller 180 may acquire an image in which the user's health care behavior is captured and the measurement information of a measurement device associated with the health care behavior, and determine whether or not the health care behavior is normally carried out based on the image or the measurement information.

The embodiment disclosed in the present disclosure may be implemented by part or combination of configurations or steps included in the foregoing embodiment. Hereinafter, the duplicate description thereof will be omitted to clearly describe the embodiment.

FIG. 5 is an exemplary view illustrating an example of a method of providing a health care service through a terminal according to the embodiment disclosed in the present disclosure.

Referring to FIG. 5, when a health care service user measures his or her blood pressure, the controller 180 may determine whether or not the subject of the health care behavior is a valid user of the health care service.

In this case, the controller 180 may acquire an image in which the user measures his or her blood pressure, and identify the subject of the health care behavior based on the acquired image.

In other words, the controller 180 may analyze the user's face region of the acquired image, and identify whether or not the user is a valid user of the health care service. Furthermore, the controller 180 may take the measurement information of the measurement device for measuring the blood pressure into consideration along with the analysis result of the image to determine whether or not the health care behavior is normally carried out. For example, when the measurement result of the blood pressure is out of a blood pressure range of the user's health history, the controller 180 may determine that blood pressure measurement is wrong since he or she is not a valid user of the health care service.

As a result of the identification, when the user is not a valid user of the health care service, the controller 180 may control an indicator indicating that the health care behavior is not normally carried out to be displayed on the screen of the terminal 100.

FIG. 6 is an exemplary view illustrating another example of a method of providing a health care service through a terminal according to the embodiment disclosed in the present disclosure.

Referring to FIG. 6, when the health care service user measures his or her blood pressure, the controller 180 may determine whether or not the measurement position of the blood pressure is correct.

In this case, the controller 180 may acquire an image in which the user measures his or her blood pressure, and determine whether or not the measurement position of the blood pressure is correct based on the acquired image.

For example, when the user tries to place a blood pressure measurement device at a wrong measurement position 220 and measure his or blood pressure during the blood pressure measurement, the controller 180 may provide guide information to the user to move the blood pressure measurement device to a correct measurement position 230 for his or her blood pressure measurement.

Furthermore, the controller 180 may take the measurement information of the measurement device for measuring the blood pressure into consideration along with the analysis result of the image to determine whether or not the health care behavior is normally carried out. For example, when the measurement result of the blood pressure is out of a blood pressure range of general people, the controller 180 may determine that the measurement is wrong due to a wrong measurement position of the blood pressure.

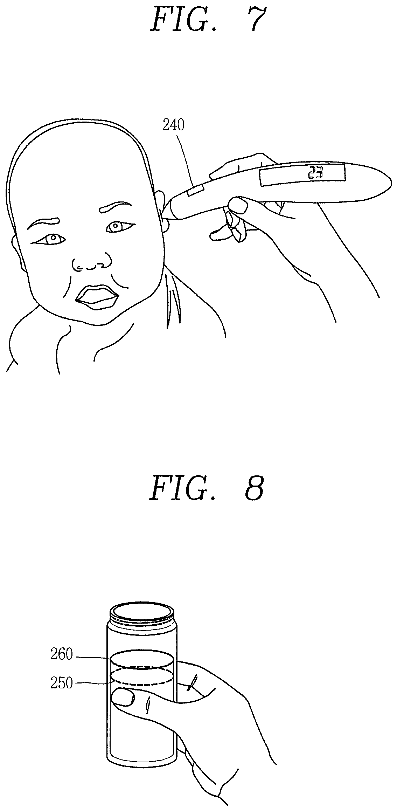

FIG. 7 is an exemplary view illustrating an exemplary method of providing a health care service through a terminal to understand the present disclosure.

Referring to FIG. 7, when a health care service user measures his or her temperature, the controller 180 may determine the operation or non-operation of a thermometer.

To this end, the controller 180 may acquire an image that has captured a scene in which the user measures his or her temperature. In this case, the controller 180 may determine the operation or non-operation of a thermometer based on the captured image. For example, in case of FIG. 7, the controller 180 may analyze a region 240 with an operation button of the thermometer from the captured image to determine whether or not the operation button of the thermometer is pressed. As a result of the determination, when the operation button of the thermometer is not pressed, the controller 180 may determine that the temperature measurement is not normally carried out.

Furthermore, the controller 180 may take the measurement information (or measurement result) of the thermometer into consideration along with the analysis result of the image to determine whether or not the temperature measurement behavior is normally carried out. For example, when the measurement result of the temperature is out of a temperature range of general people, the controller 180 may determine that the measurement is wrong due to an operational failure of the thermometer.

FIG. 8 is an exemplary view illustrating another exemplary method of providing a health care service through a terminal to understand the present disclosure.

Referring to FIG. 8, when a health care service user takes medication, the controller 180 may determine whether or not the medication dosage is normal.

To this end, the controller 180 may acquire an image capturing a scene in which the user takes medication. In this case, the controller 180 may determine the medication dosage based on the captured image. For example, in case of FIG. 8, the controller 180 may analyze a scale portion of the medication container containing medicine from the captured image to measure the medication dosage. As a result of the determination, when medicine is contained in the medication container on a marking 260 indicating an excessive dosage than on a marking 250 indicating a normal dosage, the controller 180 may determine that the user's medication behavior is not normally carried out.

Furthermore, the controller 180 may take the measurement information (for example, medication dosage) of the measurement device (not shown) mounted on the medication container into consideration along with the analysis result of the image to determine whether or not the medication behavior is normally carried out. For example, when a normal dosage is 5 cc, and an initial volume contained in the medication container and a remaining volume after administration are 8 cc and 1 cc, respectively, a dosage measured by the measurement device may be 7 cc. In this case, the controller 180 may determine that the user has administered the medicine in excess of 2 cc based on the captured image and the measured medication dosage.

Hereinafter, a method of displaying an indicator or guide information according to the embodiment disclosed in the present disclosure will be described below with reference to FIGS. 9 and 10.

According to a method of displaying an indicator or guide information in accordance with the embodiment disclosed in the present disclosure, an image in which the health care behavior is captured may be displayed on the screen of the terminal, and the indicator may be displayed at a portion where the health care behavior is abnormally carried out on the displayed screen.

The embodiment disclosed in the present disclosure may be implemented by part or combination of configurations or steps included in the foregoing embodiment. Hereinafter, the duplicate description thereof will be omitted to clearly describe the embodiment.

FIG. 9 is an exemplary view illustrating a method of displaying an indicator according to the embodiment disclosed in the present disclosure.

Referring to FIG. 9, when a health care service user measures his or her blood pressure, the controller 180 acquires an image that has captured a scene in which the user measures his or her blood pressure. Furthermore, the controller 180 analyzes the acquired image to determine whether or not the measurement position of the blood pressure is correct.

As a result of the determination, when determined that the measurement position of the blood pressure is wrong, the controller 180 controls an image in which the measurement behavior of the blood pressure is captured to be displayed on the screen of the terminal 100.

Furthermore, the controller 180 displays an indicator indicating that the measurement position of the blood pressure is abnormal at the wrong blood pressure measurement position on the displayed screen.

In case of FIG. 9, the user places a blood pressure measurement device at a wrong blood pressure measurement position to measure his or her blood pressure. In this case, an image in which the blood pressure measurement behavior is captured is displayed on the screen of the terminal 100, and the indicator 270 is displayed at the wrong blood pressure measurement position.

Furthermore, the captured image and the indicator are displayed on the screen of the terminal while carrying out the health care behavior. For example, while a health care service user performs the health care behavior, the terminal 100 acquires in which the health care behavior is captured, and determines whether or not the health care behavior is normally carried out based on the image. Furthermore, as a result of the determination, when the health care behavior is abnormally carried out, the terminal 100 displays the indicator along with the image in real time on the screen of the terminal 100. As a result, the user may confirm whether or not his or her behavior is normally carried out while performing the health care behavior, and perform the health care behavior again if it is abnormal.

Furthermore, the controller 180 determines whether or not the health care behavior is normally carried out based on an image in which the health care behavior is captured and the measurement information of the measurement device associated with the health care behavior, and provides guide information for carrying out a normal health care behavior again when the health care behavior is not normally carried out.

The provision of the guide information may be provided through at least one of an indicator, an image and a voice indicating the guide information.

FIG. 10 is an exemplary view illustrating an example of a method of displaying guide information according to an embodiment disclosed in the present disclosure.

Referring to FIG. 10, when a health care service user measures his or her blood pressure, the controller 180 acquires an image that has captured a scene in which the user measures his or her blood pressure. Furthermore, the controller 180 analyzes the acquired image to determine whether or not the measurement position of the blood pressure is correct.

As a result of the determination, when determined that the measurement position of the blood pressure is wrong, the controller 180 controls an image in which the measurement behavior of the blood pressure is captured to be displayed on the screen of the terminal 100.

Furthermore, the controller 180 provides guide information for allowing the user to measure his or her blood pressure at a correct measurement position along with the captured image.

In case of FIG. 10, the user places a blood pressure measurement device at a wrong blood pressure measurement position to measure his or her blood pressure. In this case, an image that has captured the blood pressure measurement behavior on the screen of the terminal 100 is displayed, and guide information on "Measurement position is wrong!! Please measure in a circle position of the image." Furthermore, the guide information may be provided in the form of an indicator and a voice.

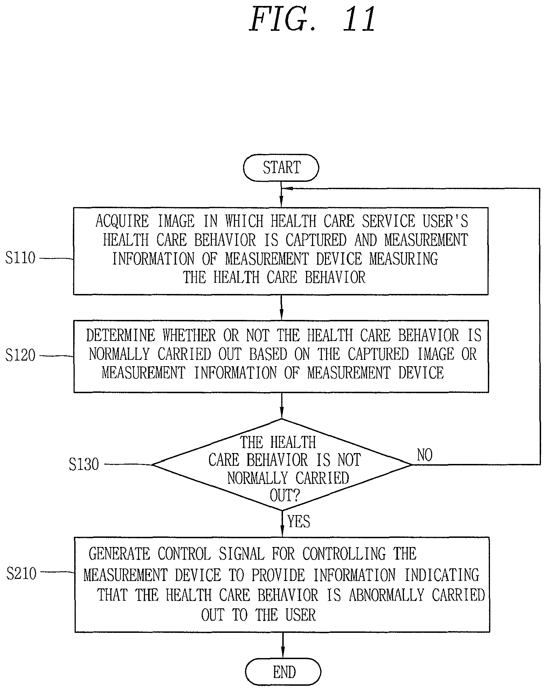

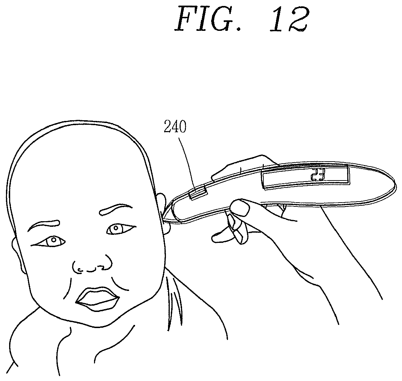

Hereinafter, a method of providing information indicating that the health care behavior through the measurement device is abnormal will be described below with reference to FIGS. 11 and 12.

A method of providing a health care service according to the embodiment disclosed in the present disclosure relates to a method of providing information indicating that the health care behavior is abnormal through a measurement device used to measure the health care behavior.

The embodiment disclosed in the present disclosure may be implemented by part or combination of configurations or steps included in the foregoing embodiment. Hereinafter, the duplicate description thereof will be omitted to clearly describe the embodiment.

According to the embodiment, the controller 180 acquires an image in which the user's health care behavior is captured and the measurement information of a measurement device associated with the health care behavior, and determines whether or not the health care behavior is normally carried out based on the image or the measurement information.

As a result of the determination, when the health care behavior is not normally carried out, the controller 180 generates a control signal for controlling the measurement device to provide information indicating that the health care behavior is abnormally carried out to the user.

Then, the controller 180 transmits the control signal to the measurement device, and the measurement device may provide information indicating that the health care behavior is abnormally carried out in response to the transmitted control signal to the user.

FIG. 11 is a flow chart illustrating a method of providing a health care service according to an embodiment disclosed in the present disclosure.

Referring to FIG. 11, a method of providing a health care service according to the embodiment disclosed in the present disclosure will be carried out with the following steps.

First, the terminal 100 acquires an image in which a health care service user's health care behavior is captured and the measurement information of a measurement device associated with the health care behavior (SI10).

Next, the terminal 100 determines whether or not the health care behavior is normally carried out based on the captured image or the measurement information of the measurement device (S120).

Next, when the health care behavior is not normally carried out (S130), the terminal 100 generates a control signal for controlling the measurement device to provide information indicating that the health care behavior is abnormally carried out to the user (S210).

Here, the information indicating that the health care behavior is abnormally carried out may be provided to the user through at least one of a vibration, an image and a voice output by means of the measurement device.

For example, the information indicating that the health care behavior is abnormally carried out may be provided with a voice through an audio output device (for example, speaker) provided in the measurement device.

FIG. 12 is an exemplary view illustrating a method of providing a health care service to understand the present disclosure.

Referring to FIG. 12, when a health care service user measures his or her temperature, the controller 180 may determine the operation or non-operation of a thermometer.

To this end, the controller 180 may acquire an image that has captured a scene in which the user measures his or her temperature. In this case, the controller 180 may determine the operation or non-operation of a thermometer based on the captured image. For example, in case of FIG. 7, the controller 180 may analyze a region 240 with an operation button of the thermometer from the captured image to determine whether or not the operation button of the thermometer is pressed. As a result of the determination, when the operation button of the thermometer is not pressed, the controller 180 may determine that the temperature measurement is not normally carried out.

Furthermore, when the temperature measurement is not normally carried out, the controller 180 may provide information indicating that the temperature measurement is not normally carried out to the user through the thermometer.

In case of FIG. 12, the controller 180 may generate a control signal for controlling the thermometer to provide information indicating that the temperature measurement is not normally carried out to the user, and the measurement device may generate a vibration output in response to the generated control signal, thereby providing information indicating that the temperature measurement is not normally carried out to the user.

A method of providing a health care service to understand the present disclosure relates to a method of transmitting at least one of the captured images and the measurement information of the measurement device to a health care server, and allowing a health care professional to check an image in which an abnormal health care behavior is captured and the measurement information through the health care server when the health care behavior is not normally carried out so as to correctly know the status of a health care service user.

In this case, the terminal 100 may feedback an advice of the health care professional, thereby allowing the user's health care behavior to be normally carried out.

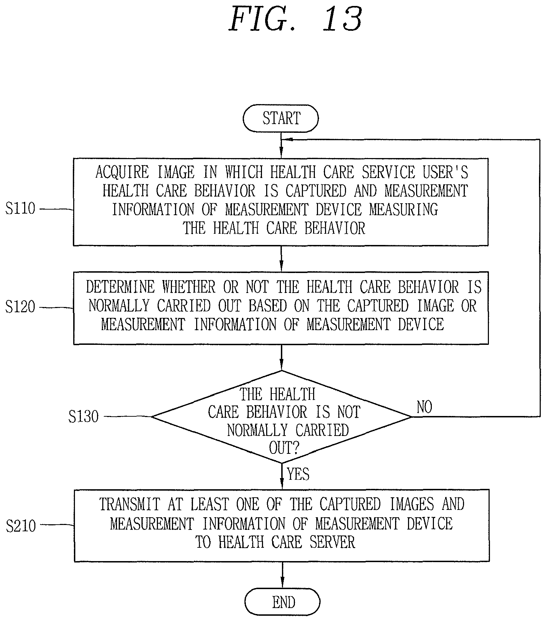

FIG. 13 is a flow chart illustrating a method of providing a health care service to understand the present disclosure.

Referring to FIG. 13, a method of providing a health care service to understand the present disclosure will be carried out with the following steps.

First, the terminal 100 may acquire an image in which a health care service user's health care behavior is captured and the measurement information of a measurement device associated with the health care behavior (S110).

Next, the terminal 100 may determine whether or not the health care behavior is normally carried out based on the captured image or the measurement information of the measurement device (S120).

Next, when the health care behavior is not normally carried out (S130), the terminal 100 may transmit at least one of the captured images and the measurement information of the measurement device to a health care server (S310).

The health care server may receive the captured image and the measurement information of the measurement device to store the captured image and the measurement information of the measurement device in a storage medium (not shown) provided in the health care server.

Furthermore, the health care professional (for example, doctor, pharmacist, etc.) may inquire the stored image and measurement information to provide guide information or the like through the terminal 100 so as to normally carry out the health care behavior.

The foregoing method may be implemented in a medium that can be read by a computer or similar device using software, hardware, or any combination thereof.

For hardware implementation, the foregoing methods may be implemented by using at least one of application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, electrical units designed to perform the functions described herein. For example, the foregoing methods may be implemented in the controller 180 of the terminal 100.

For software implementation, procedures or functions may be implemented together with separate software modules. The software modules may perform at least one function or operation described herein. Software codes can be implemented by a software application written in any suitable programming language. The software codes may be stored in the memory 160 and executed by the controller 180.

The scope of the present disclosure is not limited to the embodiment disclosed in the present specification, and thus various forms of modifications, variations, and improvements can be made in the present disclosure without departing from the concept of the invention, and within the scope of the appended claims.

## Claims

1. A method of providing a health care service through a terminal, the method comprising:
acquiring (S110) an image in which a health care service user's health care behavior is captured and measurement information of a measurement device associated with the health care behavior;
determining (S120, S130) whether or not the health care behavior is normally carried out based on the captured image by determining whether or not a position of the measurement device mounted on the user's body is correct based on the captured image;
displaying (S140) a first indicator indicating that the health care behavior is not normally carried out on a screen of the terminal when the health care behavior is not normally carried out;
displaying (S210) an image in which the health care behavior is captured on the screen of the terminal, wherein the first indicator is displayed at a position where the health care behavior is abnormally carried out on the screen, and wherein the captured image and the first indicator are displayed in real-time on the screen of the terminal while the health care behavior is carried out; and
providing guide information for carrying out a normal health care behavior, wherein the provision of the guide information is provided through a second indicator (270) indicating the correct health care behavior along with the displayed image,
wherein providing guide information further comprises, when the position of the measurement device is incorrect, generating (S210) a control signal for controlling the measurement device to provide information indicating that the health care behavior is abnormally carried out, transmitting the control signal to the measurement device, and outputting the information indicating that the health care behavior is abnormally carried out by means of the measurement device mounted on the user's body.

2. The method of claim 1, wherein the health care behavior is at least one of the user's blood glucose measuring behavior, the user's blood pressure measuring behavior, the user's eating behavior, the user's medication behavior and the user's exercise behavior.

3. The method of claim 1 or 2, wherein the measurement information of the measurement device is at least one of the user's blood glucose level, the user's blood pressure value, the user's medication dosage, the user's food intake amount, the user's workrate, the power status of the measurement device, the operation or non-operation of the measurement device, a humidity and temperature during measurement.

4. The method of any one of claims 1 to 3, wherein said determining whether or not the user's health care is normally carried out is determined based on at least one of whether or not the subject of the health care behavior is a valid user of the health care service by analysing a face region of the user of the acquired image, and the user's posture during measurement.

5. The method of any one of claims 1 to 4, further comprising:
determining whether or not the user's health care is normally carried out based on at least one of a failure or non-failure during the operation of the measurement device, and whether or not the measurement information is abnormal.

6. The method of claim 5, wherein the information indicating that the health care behavior is abnormally carried out is provided to the user through at least one of a vibration, an image and a voice output by means of the measurement device.

7. The method of any one of claims 1 to 6, further comprising:
transmitting at least one of the captured images and the measurement information of the measurement device to a health care server when the health care behavior is not normally carried out.

8. A terminal, comprising:
a display unit (151) configured to display a screen; and
a controller (180) configured to perform the method of claim 1.

9. The terminal of claim 8, wherein the health care behavior is at least one of the user's blood glucose measuring behavior, the user's blood pressure measuring behavior, the user's eating behavior, the user's medication behavior and the user's exercise behavior.

10. The terminal of any one of claims 8 to 9, wherein the measurement information of the measurement device is at least one of the user's blood glucose level, the user's blood pressure value, the user's medication dosage, the user's food intake amount, the user's workrate, the power status of the measurement device, the operation or non-operation of the measurement device, a humidity and temperature during measurement.

11. The terminal of any one of claims 8 to 10, wherein the determination of whether or not the user's health care is normally carried out is determined based on at least one of whether or not the subject of the health care behavior is a valid user of the health care service by analysing a face region of the user of the acquired image, and the user's posture during measurement, .

12. The terminal of any one of claims 8 to 11, further comprising:
determining whether or not the user's health care is normally carried out based on at least one of a failure or non-failure during the operation of the measurement device, and whether or not the measurement information is abnormal.

## Patentansprüche

1. Verfahren zum Bereitstellen eines Gesundheitspflegediensts über ein Endgerät, wobei das Verfahren umfasst:
Erfassen (S110) eines Bilds, in dem ein Gesundheitspflegeverhalten eines Benutzers eines Gesundheitspflegediensts festgehalten wird, und einer Messinformation einer mit dem Gesundheitspflegeverhalten assoziierten Messvorrichtung;
Ermitteln (S120, S130) auf Grundlage des erfassten Bilds, ob das Gesundheitspflegeverhalten normal ausgeführt wird oder nicht, durch Ermitteln auf Grundlage des erfassten Bilds, ob eine Position der am Körper des Benutzers montierten Messvorrichtung richtig ist oder nicht;
Anzeigen (S140) eines ersten Hinweises, der auf einem Bildschirm des Endgeräts darauf hinweist, dass das Gesundheitspflegeverhalten nicht normal ausgeführt wird, wenn das Gesundheitspflegeverhalten nicht normal ausgeführt wird;
Anzeigen (S210) eines Bilds, in dem das Gesundheitspflegeverhalten auf dem Bildschirm des Endgeräts erfasst wird, wobei der erste Hinweis an einer Position auf dem Bildschirm angezeigt wird, an der das Gesundheitspflegeverhalten nicht normal ausgeführt wird, und wobei das erfasste Bild und der erste Hinweis in Echtzeit auf dem Bildschirm des Endgeräts angezeigt werden, während das Gesundheitspflegeverhalten ausgeführt wird; und
Bereitstellen einer Anleitungsinformation zum Ausführen eines normalen Gesundheitspflegeverhaltens, wobei die Bereitstellung der Anleitungsinformation über einen zweiten Hinweis (270) bereitgestellt wird, der zusammen mit dem angezeigten Bild auf das richtige Gesundheitspflegeverhalten hinweist,
wobei das Bereitstellen der Anleitungsinformation, wenn die Position der Messvorrichtung falsch ist, ferner umfasst: ein Erzeugen (S210) eines Steuersignals zum Steuern der Messvorrichtung, um eine Information bereitzustellen, die anzeigt, dass das gesundheitliche Verhalten nicht normal ausgeführt wird, Senden des Steuersignals an die Messvorrichtung und Ausgeben der Information, dass das Gesundheitspflegeverhalten nicht normal ausgeführt wird, mithilfe der am Körper des Benutzers montierten Messvorrichtung.

2. Verfahren nach Anspruch 1, wobei das Gesundheitspflegeverhalten mindestens eines aus Blutzuckermessverhalten des Benutzers, Blutdruckmessverhalten des Benutzers, Essverhalten des Benutzers, Medikamentennahmeverhalten des Benutzers und Sportverhalten des Benutzers ist.

3. Verfahren nach Anspruch 1 oder 2, wobei die Messinformation der Messvorrichtung mindestens eines aus Blutzuckerspiegel des Benutzers, Blutdruckwert des Benutzers, Medikamentendosierung des Benutzers, Nahrungsaufnahmemenge des Benutzers, Arbeitsrate des Benutzers, Energiezustand der Messvorrichtung, Betrieb oder Nichtbetrieb der Messvorrichtung, Feuchtigkeit und Temperatur während der Messung ist.

4. Verfahren nach einem der Ansprüche 1 bis 3, wobei das Ermitteln, ob die Gesundheitspflege des Benutzers normal ausgeführt wird oder nicht, auf Grundlage mindestens eines aus, ob das Subjekt des Gesundheitspflegeverhaltens ein gültiger Benutzer des Gesundheitspflegediensts ist oder nicht, durch Analysieren eines Gesichtsbereichs des Benutzers des erfassten Bilds und der Körperhaltung des Benutzers während der Messung ermittelt wird.

5. Verfahren nach einem der Ansprüche 1 bis 4, ferner umfassend:
Ermitteln, ob die Gesundheitspflege des Benutzers normal ausgeführt wird, auf Grundlage mindestens eines aus einem Fehler oder fehlerfreien Zustand während des Betriebs der Messvorrichtung und ob die Messinformation nicht normal ist oder nicht.

6. Verfahren nach Anspruch 5, wobei die Information, die anzeigt, dass das gesundheitliche Verhalten nicht normal ausgeführt wird, dem Benutzer durch mindestens eines aus einer Vibration, einem Bild und einer Sprachausgabe mittels der Messvorrichtung bereitgestellt wird.

7. Verfahren nach einem der Ansprüche 1 bis 6, ferner umfassend:
Senden mindestens eines der erfassten Bilder und der Messinformation der Messvorrichtung an einen Gesundheitspflegeserver, wenn das Gesundheitspflegeverhalten nicht normal ausgeführt wird.

8. Endgerät, umfassend:
eine Anzeigeeinheit (151), die dazu eingerichtet ist, einen Bildschirm anzuzeigen; und
eine Steuerung (180), die dazu eingerichtet ist, das Verfahren nach Anspruch 1 durchzuführen.

9. Endgerät nach Anspruch 8, wobei das Gesundheitspflegeverhalten mindestens eines aus Blutzuckermessverhalten des Benutzers, Blutdruckmessverhalten des Benutzers, Essverhalten des Benutzers, Medikamentennahmeverhalten des Benutzers und Sportverhalten des Benutzers ist.

10. Endgerät nach einem der Ansprüche 8 bis 9, wobei die Messinformation der Messvorrichtung mindestens eines aus Blutzuckerspiegel des Benutzers, Blutdruckwert des Benutzers, Medikamentendosierung des Benutzers, Nahrungsaufnahmemenge des Benutzers, Arbeitsrate des Benutzers, Energiezustand der Messvorrichtung, Betrieb oder Nichtbetrieb der Messvorrichtung, Feuchtigkeit und Temperatur während der Messung ist.

11. Endgerät nach einem der Ansprüche 8 bis 10, wobei die Ermittlung, ob die Gesundheitspflege des Benutzers normal ausgeführt wird oder nicht, auf Grundlage mindestens eines aus, ob das Subjekt des Gesundheitspflegeverhaltens ein gültiger Benutzer des Gesundheitspflegediensts ist oder nicht, durch Analysieren eines Gesichtsbereichs des Benutzers des erfassten Bilds und der Körperhaltung des Benutzers während der Messung ermittelt wird.

12. Endgerät nach einem der Ansprüche 8 bis 11, ferner umfassend:
Ermitteln, ob die Gesundheitspflege des Benutzers normal ausgeführt wird, auf Grundlage mindestens eines aus einem Fehler oder fehlerfreien Zustand während des Betriebs der Messvorrichtung und ob die Messinformation nicht normal ist oder nicht.

## Revendications

1. Procédé pour fournir un service de soins de santé par l'intermédiaire d'un terminal, le procédé comprenant :
acquérir (S110) une image, dans laquelle un comportement de soins de santé d'un utilisateur de service de soins de santé est capturé, et des informations de mesure d'un dispositif de mesure associé au comportement de soins de santé ;
déterminer (S120, S130) si le comportement de soins de santé est réalisé normalement ou non sur la base de l'image capturée, par détermination quant à savoir si une position du dispositif de mesure monté sur le corps de l'utilisateur est correcte ou non sur la base de l'image capturée ;
afficher (S140) un premier indicateur indiquant que le comportement de soins de santé n'est pas réalisé normalement sur un écran du terminal lorsque le comportement de soins de santé n'est pas réalisé normalement ;
afficher (S210) une image dans laquelle le comportement de soins de santé est capturé sur l'écran du terminal, le premier indicateur étant affiché dans une position dans laquelle le comportement de soins de santé est réalisé anormalement sur l'écran, et l'image capturée et le premier indicateur étant affichés en temps réel sur l'écran du terminal tandis que le comportement de soins de santé est réalisé ; et
fournir des informations de guidage pour réaliser un comportement de soins de santé normal, la fourniture des informations de guidage étant assurée par l'intermédiaire d'un second indicateur (270) indiquant le comportement de soins de santé correct conjointement avec l'image affichée,
fournir des informations de guidage comprenant en outre, lorsque la position du dispositif de mesure est incorrecte, générer (S210) un signal de commande pour commander le dispositif de mesure pour fournir des informations indiquant que le comportement de soins de santé est réalisé anormalement, émettre le signal de commande vers le dispositif de mesure, et délivrer en sortie les informations indiquant que le comportement de soins de santé est réalisé anormalement au moyen du dispositif de mesure monté sur le corps de l'utilisateur.

2. Procédé selon la revendication 1, dans lequel le comportement de soins de santé est au moins un parmi un comportement de mesure de glycémie de l'utilisateur, un comportement de mesure de tension artérielle de l'utilisateur, un comportement d'alimentation de l'utilisateur, un comportement de médication de l'utilisateur et un comportement d'exercice de l'utilisateur.

3. Procédé selon la revendication 1 ou 2, dans lequel les informations de mesure du dispositif de mesure sont au moins un parmi un niveau de glycémie de l'utilisateur, une valeur de tension artérielle de l'utilisateur, un dosage de médicament de l'utilisateur, une quantité d'apport alimentaire de l'utilisateur, un rythme de travail de l'utilisateur, l'état d'alimentation du dispositif de mesure, le fonctionnement ou non-fonctionnement du dispositif de mesure, une humidité et une température pendant la mesure.

4. Procédé selon l'une quelconque des revendications 1 à 3, dans lequel ladite détermination du point de savoir si le soin de santé de l'utilisateur est réalisé normalement ou non est déterminée sur la base d'au moins un parmi le point de savoir si le sujet du comportement de soins de santé est ou non un utilisateur valide du service de soins de santé par analyse d'une région de visage de l'utilisateur de l'image acquise, et la posture de l'utilisateur pendant la mesure.

5. Procédé selon l'une quelconque des revendications 1 à 4, comprenant en outre :
déterminer si le soin de santé de l'utilisateur est réalisé normalement ou non sur la base d'au moins l'une parmi une défaillance ou une non-défaillance pendant le fonctionnement du dispositif de mesure, et si les informations de mesure sont anormales ou non.

6. Procédé selon la revendication 5, dans lequel les informations indiquant que le comportement de soins de santé est réalisé anormalement sont fournies à l'utilisateur par l'intermédiaire d'au moins une parmi une vibration, une image et une sortie vocale au moyen du dispositif de mesure.

7. Procédé selon l'une quelconque des revendications 1 à 6, comprenant en outre :
transmettre au moins l'une parmi les images capturées et les informations de mesure du dispositif de mesure à un serveur de soins de santé lorsque le comportement de soins de santé n'est pas réalisé normalement.

8. Terminal, comprenant :
une unité d'affichage (151) configurée pour afficher un écran ; et
un dispositif de commande (180) configuré pour mettre en oeuvre le procédé selon la revendication 1.

9. Terminal selon la revendication 8, dans lequel le comportement de soins de santé est au moins un parmi un comportement de mesure de glycémie de l'utilisateur, un comportement de mesure de tension artérielle de l'utilisateur, un comportement d'alimentation de l'utilisateur, un comportement de médication de l'utilisateur et un comportement d'exercice de l'utilisateur.

10. Terminal selon l'une quelconque des revendications 8 et 9, dans lequel les informations de mesure du dispositif de mesure sont au moins un parmi un niveau de glycémie de l'utilisateur, une valeur de tension artérielle de l'utilisateur, un dosage de médicament de l'utilisateur, une quantité d'apport alimentaire de l'utilisateur, un rythme de travail de l'utilisateur, l'état d'alimentation du dispositif de mesure, le fonctionnement ou non-fonctionnement du dispositif de mesure, une humidité et une température pendant la mesure.

11. Terminal selon l'une quelconque des revendications 8 à 10, dans lequel la détermination du point de savoir si le soin de santé de l'utilisateur est réalisé normalement ou non est déterminée sur la base d'au moins un parmi le point de savoir si le sujet du comportement de soins de santé est ou non un utilisateur valide du service de soins de santé par analyse d'une région de visage de l'utilisateur de l'image acquise, et la posture de l'utilisateur pendant la mesure.

12. Terminal selon l'une quelconque des revendications 8 à 11, comprenant en outre :
déterminer si le soin de santé de l'utilisateur est réalisé normalement ou non sur la base d'au moins une parmi une défaillance ou une non-défaillance pendant le fonctionnement du dispositif de mesure, et si les informations de mesure sont anormales ou non.
